(19) Europäisches Patentamt
European Patent Office
Office européen des brevets

(11) Publication number: **0 436 316 A1**

# (12) EUROPEAN PATENT APPLICATION

(21) Application number: **90313363.5**

(22) Date of filing: **07.12.90**

(51) Int. Cl.$^5$: **C07C 233/53,** C07C 235/16, C07C 237/46, A61K 49/04

The title of the invention has been amended (Guidelines for Examination in the EPO, A-III, 7.3).

(30) Priority: **08.12.89 US 448073**
**28.08.90 US 574300**

(43) Date of publication of application:
**10.07.91 Bulletin 91/28**

(84) Designated Contracting States:
**AT BE CH DE DK ES FR GB GR IT LI LU NL SE**

(71) Applicant: **Sovak, Milos**
**3333 North Torrey Pines Court, Suite 100**
**La Jolla, California 92037 (US)**

(72) Inventor: **Sovak, Milos**
**3333 North Torrey Pines Court, Suite 100**
**La Jolla, California 92037 (US)**

(74) Representative: **Harrison, David Christopher et al**
**MEWBURN ELLIS 2 Cursitor Street**
**London EC4A 1BQ (GB)**

## (54) X-ray contrast media for prolonged opacification.

(57) Hydroxyalkyl amino-substituted triiodobenzoates are provided, where the remaining position is substituted by amino or carboxy. The compounds have low solubility in the gastrointestinal tract, but are resorbable from extravisceral body cavities. They can be readily formulated and used as contract media for the plain radiography of the GI tract.

Also, addition polymers comprised of triiodo compounds bonded through an amino nitrogen to a non-oxo-carbonyl group of an addition polymerizable monomer are provided. The polymers are highly water soluble physiologically acceptable, and not resorbable from the GI tract, but from the extravisceral body cavities. The polymers find use for computer tomography of the GI tract.

EP 0 436 316 A1

# X-RAY CONTRAST MATERIALS FOR THE GASTROINTESTINAL TRACT

The field of this invention concerns contrast media for the examination of the gastrointestinal tract by means of x-ray.

The x-ray is the most often-used diagnostic tool for the examination of the gastrointestinal tract, i.e., the pharynx, esophagus, stomach, duodenum, small bowel, and colon. While fibre-optics and other forms of direct examination are used to some extent, x-ray examinations of the GI tract, with either plain film radiography or radiography assisted by x-ray computerized tomography (CT), prevail. Because of the low inherent contrast differential within the tissues, these examinations must be performed with contrast media administered into the tract. None of the media now available are ideal for this purpose.

The ideal contrast medium for GI application would be completely biologically inert, both in the tract and in the surrounding tissues and cavities, where it can leak out through a perforation. It should not affect the fluid balance within the tract. It should be able to delineate anatomical detail accurately and consistently in all patients, provide adequate radio-pacity, and be homogeneously distributed among the mucosal folds. Beyond this, there are specific properties crucial for applications in each of the two main examining modalities.

For plain film radiography the contrast medium must have a very high iodine content, i.e., at least 50% iodine weight per volume. With water soluble compounds, this has been possible only at high concentrations, which in turn have high osmolality and thus perturb the fluid balance. The use of stable water insoluble micro-granular suspensions avoids the problem, but these should be excretable and non-toxic., The suspension should be unaffected by pH and viscosity changes throughout the tract, and it should have no osmotic effects. The medium should not change the state of the tract and readily adhere to the tract mucosa to depict minute anatomical detail, have adequately low viscosity and sufficient opacity, and should not flocculate in the fluids of the GI tract. In addition, its production should be economical.

For x-ray computerized tomography (CT), the requirements for a contrast medium are essentially the same as stated above except that the medium's diagnostically useful iodine levels should be around 10 mg I/ml. Also, a CT contrast agent must be absolutely homogeneous to avoid imaging artifacts which are known to invalidate the diagnostic results. To that end, solutions rather than suspensions have to be used. While the iodine-containing compound must be water soluble, it must be inert, and not absorbable from the GI tract. In addition, such compounds, if displaced into the peritoneal cavity by a pathological perforation within the tract, must not cause an adverse reaction.

At this time, either barium sulfate or water-soluble urovascular contrast media are employed. Both media classes have many inadequacies, and may be hazardous to the patient. The barium sulfate suspension is too opaque and non-homogenous for CT, and even in plain radiography it has a tendency to cause intestinal impaction ; it does not delineate the mucosa in the distal part of the GI tract when the contents have not been completely voided. In the event of a perforation, the barium sulfate spilled into the abdomen results in barium peritonitis, which reportedly has about a 50% mortality rate. The highly toxic barium sulfate causes chemical injury to the peritoneal surface and perturbs vascular permeability, which can result in barium particles entering the capillaries and potentiating systemic toxicity. Barium sulfate, therefore, has many shortcomings, highlighting a substantial need for the development of more suitable contrast materials.

Water-soluble contrast media are used when a perforation of the GI tract or a clinically manifest bowel obstruction is suspected. Whether ionic or nonionic, such media are hyperosmolal vis-a-vis the tract and have numerous other disadvantages. They draw fluids into the intestinal lumen, thus distending the bowel wall and obscuring mucosal detail. They also act as laxatives, causing diarrhea which can possibly threaten the health of dehydrated older patients, children and infants. Furthermore, all these media have an extremely unpleasant taste, and whether used for plain radiography or CT, have to be administered in large doses of many hundreds of milliliters. The nonionic contrast media are also not adequate because of their hyperosmolality vis-a-vis the tract, and furthermore are prohibitively expensive. There is therefore, a serious need for new contrast materials with none, or fewer, of the shortcomings of the presently available media.

U.S. Patent No. 3,199,858 describes hydroxyalkyl esters of triiodoaminobenzoic acids. EPO 300,828 describes metabolically labile or water-insoluble esters to be administered intravenously as microparticles for uptake in the reticuloendothelial system. U.S. Patent No. 3,795,698 describes various esters reported to have use as x-ray contrast agents. U.S. Patent No. 4,225,725 reports esters of triiodobenzoic acid, particularly ethoxymethyl esters of acretrizoic, iothalamic and iodobenzamic acids. U.S. Patent No. 4,044,048 describes polyalkyleneoxy esters of tetraiodoterephthalic acid as x-ray contrast agents. EPA 0300828 describes water soluble iodinated aryl esters for use as contrast material. See also DE-A-2,428,140 ; GB-A-2,157,283 Chemical Abstracts 79 : 33, 1973, Abstract No. 42198G, and 84 : 39, 1976, Abstract No. 797318. Czechoslovak patent Nos. 231699 and 207858 describe grafting of triiodobenzoic acid derivatives onto various existing polymers.

Brown et al., Bull. Soc. Chim. de France (1986) 669-677, describes a polyhydroxyalkyl substituted triiodo aminoisophthaldiamide, Brown et al (Makromol Chem. Rapid Commun., (1985) 6, 503), describes a polyacrylamide of a substituted triiodo aminobenzoate.

Derivatives of triiodobenzoic acid are provided where the derivatives are characterized by being of low toxicity, useful as gastrointestinal x-ray contrast media and the aryl group has an acylated amino group and a carboxyl group. The derivatives may have a single triiodobenzoic acid or a plurality of triiodobenzoic acids, particularly polymers having at least 50 kDal molecular weight.

The monomeric compounds are hydroxyalkyl esters of limited water solubility, particularly derived from existing triiodinated organic acids used as contrast media. Particularly, alkyl or hydroxyalkyl esters of triiodobenzoic acid are provided, where the remaining two positions are occupied by at least one acylated amino group, and any remaining position by carbamoyl, wherein the nitrogen atoms may be substituted with alkyl or hydroxyalkyl. The compound has at least two hydroxyl groups. The subject compositions have limited solubility in the gastrointestinal tract, but are resorbable from extravisceral cavities.

Also provided are highly water-soluble high molecular weight copolymers containing fully substituted triiodinated moieties, acrylamides, or hydroxyalkyl acrylamides, and in some cases alkyl or hydroxyalkyl diacrylates. They find use as safe contrast media in oral or rectal application. The monomer suspensions are useful in plain x-ray radiography ; the polymer solutions are useful in x-ray computerized tomography.

Compositions, formulations and methods are provided for detecting surfaces by plain radiography and x-ray computerized tomography (CT). These compounds share common features in having triiodobenzene rings, an acylated amino group, and the remaining positions have at least one non-oxo-carboxyl group, conveniently a carboxylic acid, and, as appropriate, an amino group. For the most part, the compounds will have the following formula :

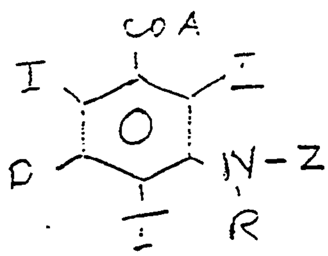

wherein :

Z is acetyl, an addition polymerizable acrylic acid group, which may be substituted or unsubstituted, or a monomer unit of a polyacrylic acid polymer ;

the definition of A and D will vary with the definition of Z,

where A is hydroxy, amino or substituted derivatives thereof of from 0 to 12 carbon atoms ; and

D is non-oxo-carboxyl, amino or substituted amino,

where the monomeric compound or unit will have at least 9 carbon atoms and not more than about 26 carbon atoms.

For the most part, the symbols will have the following definitions :

Z is acetyl, an addition polymerizable acrylyl group, or a monomeric unit of an acrylyl polymer, wherein said acrylyl group has from 0 to 1 substituent at the α carbon atom, said substituent being alkyl, hydroxyalkyl, carboxamidoalkyl, carboxyalkyl or a monomeric acrylyl group, said substituent being of from 1 to 5 carbon atoms ;

when Z is acetyl :

R is hydrogen, lower alkyl of from 1 to 6 carbon atoms or hydroxy- or polyhydroxyalkyl of from 2 to 6 carbon atoms ;

D is —NR'$COCH_3$, $CONHCH_2CH_2OH$ or $CONHCH_3$ ; and

R and R' are H, alkyl of from 1 to 6 carbon atoms or hydroxy- or polyhydroxyalkyl of from 2 to 6 carbon atoms, wherein R and R' may be same or different ;

A is alkoxy of from 1 to 4 carbon atoms or hydroxy- or polyhydroxyalkyl of from 2 to 4 carbon atoms ;

when Z is an addition polymerizable acrylyl group or a monomeric unit of an acrylyl polymer ;

R is of 0 to 4 carbon atoms and is hydrogen, alkyl or hydroxyalkyl of from 1 to 3 hydroxyl groups ;

A is hydroxyl, O-M, where M is a physiologically acceptable counter ion or carboxamide, where the nitrogen may have from 0 to 2 substituents, which substituents are alkyl of from 1 to 4 carbon atoms, hydroxyalkyl of from 2 to 4 carbon atoms and 1 to 3 hydroxyl groups, any remaining positions being occupied by hydrogen ;

D is a non-oxo-carbonyl group or amino group, wherein said non-oxo-carbonyl group is a carboxylic acid, a physiologically acceptable salt, or a carboxamide, wherein the nitrogen has from 0 to 2 substituents, which substituents are alkyl of from 1 to 4 carbon atoms or hydroxyalkyl of from 2 to 4 carbon atoms and 1 to 2 hydroxyl groups, any remaining positions being occupied by hydrogen.

For plain radiography, hydroxyalkyl esters of aromatic organic acids which previously have found use as salts or amides as contrast media or analogs thereof are used. Particularly, polyiodinated benzoates are provided of limited solubility in the gastrointestinal tract. The compounds are characterized by having a benzene ring, three symmetrically substituted iodines, with the remaining positions occupied by carboxamide or amino, where the nitrogens are substituted by alkyl, hydroxyalkyl, or acyl. The nitrogens may be di- or trisubstituted, with the substituents on the various groups being the same or different. The compounds will normally have at least about 13 carbon atoms, usually from about 15 to 20 carbon atoms and from about 2 to 8 hydroxyl groups, usually 2 to 6 hydroxyl groups, preferably from about 2 to 4 hydroxyl groups.

For the most part, the compounds of the subject invention will have the following formula :

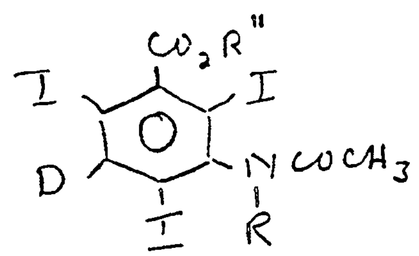

wherein :

the R″ group is lower alkyl of 1 to 6, usually 1 to 4 carbon atoms, particularly methyl, hydroxy- or polyhydroxyalkyl of from 2 to 6, particularly 2 to 4 carbon atoms and 1 to 5, particularly 1 to 3 hydroxyl groups, more particularly -2,3,4, or 1,3,4- trihydroxybutyl, dihydroxypropyl, either 2,3- or 1,3-, preferably 2,3-, or 2-hydroxyethyl, more usually R″ will not be more than 3 carbon atoms ;

D is —$NR'COCH_3$, $CONHCH_2CH_2OH$ or $CONHCH_3$ ; and

R and R′ are H, alkyl of from 1 to 4 carbon atoms or hydroxy- or polyhydroxyalkyl of from 2 to 4 carbon atoms, wherein R, R′ and R″ may be same or different.

The subject monomeric compounds will have limited water solubility at 37°C, generally having less than about 50% water solubility, usually less than about 20% water solubility, generally having in the range of about 2 to 30%, where water solubility is defined as the percentage of 0.66 mMols of the subject composition dissolved in water at 37°C at pH 2 and/or 8, at 4.5 hours, at equilibrium from 0.4 mols per liter of the subject composition suspended in the water. Solubility will generally range to 2 to 40%, usually 3 to 35%.

The compounds are found to be resorbed within about 24 to 48 hours after injection intraperitoneally and upon histological examination, the peritoneum shows no sign of adverse reaction. Upon resorption by the peritoneum, the subject compounds are taken into the circulation and excreted like other radiographic contrast media into the urine. The ester is not appreciably hydrolyzed during the time the subject compounds are present in the host.

The compounds can be readily prepared by relatively simple procedural steps. A number of triiodinated starting materials are available, such as metrizoate, diatrizoate, iothalamate and ioxithalamate. Therefore, due to the commercial availability of the starting materials, the synthesis requires relatively few steps. The order of substitution is not critical, although depending upon the nature of the starting material, one order may be preferred over another. In addition, in some instances, disubstitution may be achieved in a single step, particularly as to O- and N-alkylation. Conveniently, O-alkylation may be carried out under mild conditions at mildly elevated temperatures, using a polar organic solvent. N-alkylation may be achieved using an alkali metal alkoxide and haloalkanol, particularly chloroalkanol, in a polar organic solvent, conveniently an alkanol. Where different hydroxyalkyl groups are to be present on oxygen and nitrogen, nitrogen substitution may be achieved by first alkylating the carboxyl group, e.g., methylation, followed by N-alkylation as described previously. For "one step" O and N-alkylation the conditions employed are a polar protic or aprotic solvent, initially low pH (less than 7), followed by increase of pH when O-alkylation is complete.

The subject compositions may be formulated in accordance with conventional ways for use in the gastrointestinal tract. For oral use in conjunction with standard or computerized radiography, particles are prepared by combining the subject compound with a granulating or pelleting composition, such as guar, pectin, gelatin and the like. Normally from about 1 to 5 parts of the granulating composition will be employed per 10 parts of the

subject compound. In addition, other components of the particles which may be included are a sugar, such as lactose, fructose and others, where the sugar serves to bind the components and to improve the taste. The sugar will generally be from about 0.1 to 1 part per part of the subject composition, usually from about 0.3 to 0.7 parts. Other minor ingredients which may be included are citric acid, in from about 0.01 to 0.1 part, and various fragrances. The ingredients are combined, thoroughly mixed and granulated to provide particles of an acceptable size for introduction into the GI tract, generally being on the average within the range of about 0.1 to 0.5 mm, preferably about 0.2 to 0.3 mm.

For conventional plain radiography, for oral use, suspensions or solutions in water are prepared either in advance or ad hoc from a mixture of the subject compound with lactose, citric acid, methylcellulose, and a detergent, particularly a non-ionic detergent such as Tween-80. For intestinal (rectal) use, only methylcellulose (or other polysaccharide) and a detergent are used. The proportions of the components will come within the above limitations, where the detergent will be present in from about 0.1 to 1 weight percent of the composition (excluding water).

For computerized x-ray tomography, polymers or copolymers containing residues of triiodinated benzoic acid acrylamide derivatives, acryl groups, hydroxyalkylacryl groups, and/or diacryl groups are provided. These new copolymers are compounds of high hydrophilicity and water solubility, although they have high molecular weight, varying from 50,000 to several million, with a molecular weight between 300,000, and 1,000,000 daltons preferred. The iodinated benzoic acid derivatives are characterized by having a benzene ring, three symmetrically substituted iodines, and the three remaining positions are substituted either with one non-oxo-carbonyl (carboxy) and two amino or one amino and two non-oxo-carbonyl groups. Carboxamides where the nitrogens are substituted by hydroxyalkyls or acyls are also acceptable with one amino group substituted with acrylic acid. This acrylic acid residue then serves for direct incorporation of the benzoate or derivative thereof into the polyacrylamide copolymer. Various acrylamides, such as alkyl and hydroxyalkyl substituted acrylamides can be used. The polyacrylamide chain may also contain various diacryl moieties, linked by a biodegradable linker. For example, components such as the residue of ethylene glycol diacrylate and related diacrylates provide ester bonds, which may be amenable to hydrolysis and/or enzymatic attack by esterases. Bis-gem-acrylamides such as N, N'-methylene bis acrylamide may also be used to provide biodegradable links. Quantities of the hydrolyzable components are used to result in copolymers, which once hydrolyzed would provide fragments of a smaller molecular weight than 50,000 Daltons. Such fragments are readily excretable, even from the cardiovascular system.

The polymers will be derived from the following triiodinated monomer :

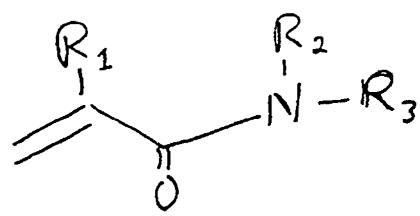

wherein :

$R_1$ is hydrogen, alkyl, hydroxyalkyl, carboxamidoalkyl, carboxyalkyl or a monomeric acrylyl group, said substituent being of from 1 to 6, usually 1 to 5, more usually 1 to 3, carbon atoms ;

$R_2$ is hydrogen, alkyl of from 1 to 3 carbon atoms or hydroxyalkyl of from 2 to 3 carbon atoms and 1 to 2 hydroxyl groups ;

$R_3$ is of the formula :

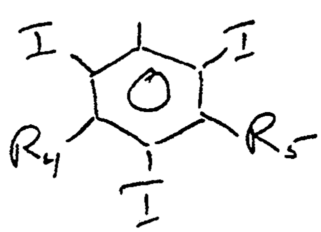

wherein :

$R_4$ and $R_5$ are the same or different and are non-oxo-carbonyl or amino, at least one being non-oxo-carbonyl, where the non-oxo-carbonyl group is a carboxylic acid, its physiologically acceptable salt or carboxamide, wherein the nitrogen has from 0 to 2 substituents, which substituents are alkyl of from 1 to 4 carbon atoms or hydroxyalkyl of from 2 to 4 carbon atoms and 1 to 3 hydroxyl groups.

The monomer will usually have at least 10 carbon atoms, usually at least 11 carbon atoms, more usually at least 12 carbon atoms and usually not more than 25 carbon atoms, more usually not more than about 16 carbon atoms. Usually, there will be at least one carboxylic acid group, although this will vary with the comonomers.

Usually, copolymers will be employed, where the other comonomer will be

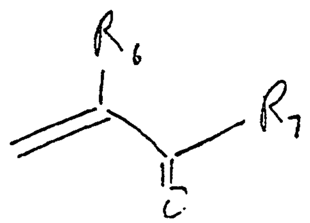

wherein :

$R_7$ is hydroxyl or a physiologically acceptable salt thereof, amino or substituted amino, wherein the substituents are alkyl of from 1 to 3 carbon atoms or hydroxyalkyl of from 2 to 3 carbon atoms and 1 to 2 hydroxyl groups, of from 1 to 3 carbon atoms or hydroxyalkoxy of from 2 to 3 carbon atoms and 1 to 2 hydroxyl groups ;

$R_6$ is hydrogen, alkyl of from 1 to 3 carbon atoms, or hydroxyalkyl of from 2 to 3 carbon atoms and 1 to 2 hydroxyl groups,

where the comonomer may vary, $R_6$ usually being hydrogen, where from about 0 to 30% of the comonomer may have $R_6$ other than hydrogen.

Desirably a cross-linking agent is used having a physiologically labile link joining the addition polymerizable groups. For the most part, these compounds will come within the following formula :

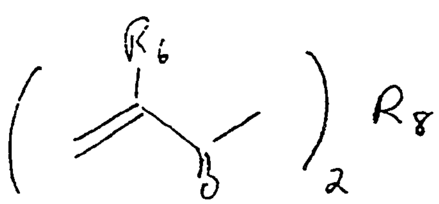

wherein :

$R_6$ has been defined previously ; and $R_8$ is a bis-functional moiety of 0 to 4 carbon atoms, which may be oxygen, nitrogen, sulfur, alkylene of from 1 to 4, usually 1 to 3 carbon atoms, or substituted alkylene, usually hydroxyl substituted of from 1 to 3 carbon atoms, where the alkylene is joined to the carbonyl group through a heteroatom, normally oxygen, nitrogen or sulfur.

The comonomer will normally be present in a mole ratio of about 10 - 50 : 1 to the triiodoaryl containing monomer, while the cross-linking monomer, if present will be in a ratio of about 0.05 - 0.5 : 1 to the triiodoaryl containing monomer. The amount of cross-linking agent should allow for a reduction in molecular weight to fragments under 75 kDal, preferably under about 50 kDal.

The molecular weight of the polymer will usually be at least about 10 kDal (weight average), more usually at least about 50 kDal, and when cross-linked may be 1,000 kDal or more.

Illustrative substituents include methyl, ethyl, propyl, 2-hydroxypropyl, 2-hydroxyethyl, 2,3-dihydroxypropyl, hydroxyacetyl, 2-3-dihydroxypropionyl, and the like, where the substituents may be bonded to carbon, nitrogen or oxygen. Cross-linking groups include methylene, *bis*-diaminomethylene, 1,2-dihydroxyethylene,, 1,3-diamino-2-hydroxypropylene, and imino.

The polymer may be prepared by any convenient means, conveniently being a random copolymer, but special techniques may be used to provide for block copolymers, or other ordered arrangement.

For x-ray CT, the solution concentration of iodine should be 5 to 15 mg/ml, preferably 10 mg/ml ; for plain radiography, about 400 to 800 mg I/ml of suspension is needed, preferably 600 mg I/ml. Generally, the fractions of the subject compounds in water will be from abut 0.5 to 150% by weight/volume ; for standard radiography

will be about 20 to 150% by weight/volume. The viscosity of the compositions for administration will be in the range of about 5 cps to 5000 cps, with the preferred range from 10 to 1000 cps. for the suspension, and 1 to 10 cps. for solutions.

The following examples are offered by way of illustration and not by way of limitation.

## EXPERIMENTAL

### EXAMPLE 1

O-Alkylation of Sodium Diatrizoate (1) to : 2-hydroxyethyl 3,5-diacetamido-2,4,6-triiodobenzoate (2)

Sodium diatrizoate (1) (30 g, 0.047 moles) was dissolved in 70 ml dimethyl sulfoxide at 50°C. 2-chloroethanol (5.12 g, 0.0636 moles) was added and the reaction temperature was raised at 70°C. After 72 hours at 70°C, the reaction was complete as indicated by TLC.

Water (150 ml) was added and a white precipitate was filtered, rinsed with $H_2O$ (30 ml x 2) and dried in vacuo to yield 26.53 grams ((2), 86% yield, 99% pure by HPLC).

### EXAMPLE 2

N-Alkylation of 2-hydroxyethyl 3,5-diacetamido-2,4,6-triiodobenzoate (2) to : 2-hydroxyethyl 3,5-{N,N'(2-hydroxyethyl)diacetamido}-2,4-6-triiodobenzoate (3)

The title compound (2) (20 g, 0.0304 moles) was suspended in methanol (120 ml) at 40°C, and 4.6 molal sodium methoxide (3.3 g, 0.0152 moles), trisodium phosphate dodecahydrate (46.22 g,, 0.1216 moles), and 2-chloroethanol (6.61 g, 0.0821 moles) were added. Further 4.6 molal sodium methoxide (14.82 g, 0.0684 moles) and 2-chloroethanol (1.22 g, 0.0152 moles) were added over a period of 29 hours to complete the reaction.

The insoluble salts were filtered off, rinsed with methanol (20 ml x 2), and the filtrate was neutralized with concentrated HC1 to pH 7. The resulting sodium chloride was filtered, the filtrate reduced to an oil and diluted with methanol (30 ml) and $H_2O$ (100 ml) to give, after 15 hours, a white crystalline solid. The mixture was filtered, rinsed with $H_2O$ (10 ml x 2) and dried to yield 14.38 g ((3), 63% yield, 97-98% pure).

### EXAMPLE 3

O-Alkylation of Sodium Diatrizoate (1) to : 2,3-dihydroxypropyl 3,5-diacetamido-2,4,6-triiodobenzoate (4)

Sodium diatrizoate (1) (30 g, 0.047 moles) was dissolved in dimethyl sulfoxide (70 ml) at 50°C. 3-Chloro-1,2-propanediol (7.03 g, 0.0636 moles) was added, the reaction temperature was raised to 100°C. The reaction was complete by TLC after 72 hours at 100°C.

The solvent was distilled off in vacuo and $H_2O$ (300 ml) was added to the resulting oil to yield a solid which was filtered and dried to give 26.3 grams ((4), 81% yield, 99% pure by HPLC).

### EXAMPLE 4

N-Alkylation of 2,3-dihydroxypropyl 3,5-diacetamido-2,4,6-triiodobenzoate (4) to : 2,3-dihydroxypropyl 3,5-{N,N' -(2-hydroxyethyl)diacetamido}-2,4,6-triiodobenzoate (5)

The title compound (4) (20 g, 0.0291 moles) was suspended in methanol (120 ml) at 40°C, and 4.6 molal sodium methoxide (3.16 g, 0.0146 moles), trisodium phosphate dodecahydrate (44.2 g, 0.116 moles) and 2-chloroethanol (6.342 g, 0.0785 moles) were added. Futher sodium methoxide (14.22 g, 0.065 moles) and 2-chloroethanol (2.34 g, 0.0291 moles) were added over the next 66 hours by which time TLC indicated reaction completion.

The insoluble salts were filtered off, rinsed with methanol (20 ml x 2) and the filtrate was neutralized with concentrated HCl. The resulting sodium chloride was filtered off, and the filtrate was evaporated to a foam to which $H_2O$ (50 ml) was added. After 24 hours, a white crystalline solid was filtered, rinsed with $H_2O$ (10 ml x 2), and dried in vacuo to yield 14.72 g ((5), 65% yield, 94-95% pure by HPLC).

### EXAMPLE 5

O-Alkylation of Sodium diatrizoate (1) to : Methyl 3,5-diacetamido-2,4,6-triiodobenzoate (6) :

Sodium diatrizoate (1) (30 g, 0.047 moles) was dissolved in 70 ml of dimethyl sulfoxide at 40°C, and methyl iodide (9.04 g, 0.0637 moles) was added. After 1 hour at 40°C, TLC indicated completion of the reaction.

Water (450 ml) was added to precipitate a white solid which was filtered off, rinsed with $H_2O$ (30 ml x 2) and dried in vacuo to yield 25.5 g ((6), 86% yield, 98% pure by HPLC).

EXAMPLE 6

N-Alkylation of methyl 3,5-diacetamido-2,4,6-triiodobenzoate (6) to : Methyl 3,5-{N,N'-(2,3-dihydroxypropyl)diacetamido}-2,4,6-triiodobenzoate (7)

The title compound (6) (20 g, 0.0319 moles) was suspended in methanol (120 ml) at 40°C. 4.6 molal sodium methoxide (3.46 g, 0.016 moles), trisodium phosphate dodecahydrate (48.43 g, 0.127 moles) were added. Over 24 hours, sodium methoxide (3.46 g, 0.016 moles) and 3-chloro-1,2-propanediol (1.76, 0.016 moles) were added to achieve reaction completion as determined by TLC.

After 48 hours, the insoluble salts were filtered off, rinsed with methanol (20 ml x 2) and the filtrate was neutralized with concentrated HC1. The resulting sodium chloride was filtered off, and the filtrate was evaporated to an oil to which $H_2O$ (50 ml) was added to give crystals which are filtered and dried to yield 5.17 g (7) (96% pure). HPLC indicated this solid was one isomer ; the other, water soluble isomers were obtained by evaporation to give an 84% total yield.

EXAMPLE 7

O-Alkylation of Diatrizoic acid (1) to : 1,3,4-trihydroxybutyl 3,5-diacetamido-2,4,6-triiodobenzoate (8)

Diatrizoic acid (1, 15.0 g, 24.4 mmoles) was dissolved in dry N,N-dimethylacetamide (50 ml) and 2,3-epoxy-1,4-butanediol (3.30 g, 31.78 mmoles) was added initially, followed by 0.51 g (4.88 mmoles) over 48 hours, during which the solution was stirred at 90°C.

The solvent was distilled off and the product was precipitated by 1-pentanol (100 ml). The solid was filtered, washed with 1-pentanol (25 ml x 2), vacuum dried, dissolved in hot water (50 ml) and stirred until complete crystallization. Filtration, washing with cold water (15 ml x 2) and drying gave an off-white solid (8) (11.75 g, 67% yield).

EXAMPLE 8

O-Alkylation of sodium iothalamate (9) to : 2,3-dihydroxypropyl 5-acetamido-2,4,6-triiodo-3-{N-(methyl)-carbamoyl}benzoate (10)

The title compound (9) (40.00 g, 0.063 moles) was dissolved in dimethyl sulfoxide (100 ml) and heated to 90°C. 3-Chloro-1,2-propanediol (9.78 g, 0.089 moles) was added and the reaction mixture was heated at 100°C for 48 hours when TLC indicated completion. Removal of the solvent under high vacuum gave a thin oil (ca. 50 ml) to which $H_2O$ (100 ml) was added. The product crystallized after 48 hours and filteration and drying gave a white powder, ((10), 36.50 g, 0.053 moles, 84% yield).

EXAMPLE 9

N-Alkylation of 2,3-dihydroxypropyl 5-acetamido-2,4,6-triiodo-3-{N-(methyl) carbamoyl}benzoate (10) to : 2,3-dihydroxypropyl 5-{N-2,3-dihydroxypropyl)-acetamido}-2,4,6-triiodo-3-{N-(methyl) carbamoyl}-benzoate (11)

The title compound (10) (30.27 g, 0.044 moles) was dissolved in methanol (150 ml) at 40°C, and sodium methoxide (25% w/w, 4.70 g, 0.022 moles), trisodium phosphate dodecahydrate (41.80 g, 0.110 moles) and 3-chloro-1,2-propanediol (9.73 g, 0.088 moles) were added. After 20 hours at 40°C, more sodium methoxide (25% w/w, 4.70 g, 0.022 moles) and 3-chloro-1,2-propanediol (2.40 g, 0.022 moles) were added. The reaction was complete after 46 hours when HPLC showed 96% conversion to product.

The insoluble phosphate salts were filtered and washed with methanol (50 ml x 2) and the filtrate was neutralized with 12 N hydrochloric acid. The resulting sodium chloride was filtered, washed the methanol (25 ml x

2) and the filtrate was concentrated in vacuo to 45 ml. Water (80 ml) was added and the product crystallized after 48 hours. The crystals were filtered, washed with cold $H_2O$ (25 ml × 2) and dried to give ((11), 20.1 g, 0.026 moles, 59% yield, 98.1% pure).

EXAMPLE 10

O-Alkylation of sodium ioxithalamate (12) to : 2-hydroxyethyl 5-acetamido-2,4,6-triiodo-3-{N-(2-hydroxyethyl) carbamoyl}benzoate (13)

The title compound (12) (40.00 g, 0.060 moles) was dissolved in dimethylsulfoxide (100 ml) and heated to 90°C. 2-Chloroethanol (5.62 ml, 0.084 moles) was added and the reaction mixture was heated at 100° for 48 hours when TLC indicated completion. Removal of the solvent under high vacuum gave a thin oil (ca. 50 ml), to which $H_2O$ (100 ml) was added. The product crystallized after 48 hours and filtration and drying gave a white powder ((13, 35.4 g, 0.051 moles, 85% yield).

EXAMPLE 11

N-Alkylation of 2-hydroxyethyl 5-acetamido-2,4,6-triiodo-3-{N-(2-hydroxyethyl) carbamoyl}benzoate (13) to : 2-hydroxyethyl 5-{N-(2-hydroxyethyl)-acetamido]-2,4,6-triiodo-3-{N-(2-hydroxyethyl) carbamoyl}benzoate (14)

The title compound (13) (30.00 g, 0.044 moles) was dissolved in methanol (150 ml) at 40°C, and sodium methoxide (25% w/w, 4.70 g, 0.22 moles), trisodium phosphate dodecahydrate (41.8 g, 0.110 moles) and 2-chloroethanol (5.90 ml, 0.088 moles) were added. After 15 hours at 40°C, more sodium methoxide (25% w/w, 4.7 g, 0.022 moles) and 2-chloroethanol (1.5 ml, 0.022 moles) were added. The reaction was complete after 51 hours when HPLC showed 95% conversion to product.

The insoluble phosphate salts were filtered, washed with methanol (50 ml x 2) and the filtrate was neutralized with 12 N hydrochloric acid. The resulting sodium chloride was filtered, washed with methanol (25 ml x 2) and the filtrate was concentrated *in vacuo* to 40 ml. Water (75 ml) was added and the product crystallized after 48 hours. The crystals were filtered, washed with cold $H_2O$ (25 ml x 2) and dried to give ((14), 21.5 g, 0.029 moles, 67% yield, 98.5% pure).

EXAMPLE 12

O- and N-Alkylation of sodium metrizoate (15) to : 2,3-dihydroxypropyl 5-{N-methyl acetamido}-2,4,6-triiodo-3-{N-(2,3-dihydroxypropyl) acetamido] benzoate (16)

The title compound (15) (50.00 g, 0.77 moles) was dissolved in dimethylsulfoxide (125 ml) and heated to 85°C. 3-Chloro-1,2-propanediol (11.5 g, 0.104 moles) was added and the reaction mixture was heated at 100°C for 24 hours when TLC indicated O-alkylation to be complete. Removal of the solvent under high vacuum gave a thick brown oil (ca. 50 ml).

The brown oil (ca. 50 ml, 0.077 moles) was dissolved in methanol (100 ml) at 40°C, and trisodium phosphate dodecahydrate (73.2 g, 0.193 moles) and 3-chloro-1,2-propanediol (17.02 g, 0.154 moles) were added. The reaction was complete after 44 hours when HPLC showed 93% conversion to product.

The insoluble phosphate salts were filtered, washed with methanol (50 ml x 2) and the filtrate was neutralized with 12^N hydrochloric acid. The resulting sodium chloride was filtered, washed with methanol (25 ml x 2) and the filtrate was concentrated *in vacuo* to 40 ml. Water (150 ml) was added and the product crystallized after 48 hours. The crystals were filtered, washed with cold $H_2O$ (25 ml x 2) and dried to give ((16), 34.7 g, 0.045 moles, 58% yield, 97.5% pure.)

EXAMPLE 13

Oral Conventional (Plain) Radiography

140 grams of one of the claimed monomeric compounds of this invention is mixed with 15 grams of lactose, 2 grams of citric acid, 1 gram of methycellulose and 1 ml of Tween-80. The ingredients are mechanically mixed.

Prior to use, water is added to obtain a suspension of desired viscosity which is conventional for convenient administration, and the mixture is administered.

EXAMPLE 14

Rectal (Intestinal) Conventional (Plain) Radiography

100 grams of one of the claimed monomeric compounds of this invention is mixed with 1 ml Tween-80 and 1 gram of methycellulose. The ingredients are mechanically mixed. Prior to the use, water is added to obtain a suspension of desired viscosity.

TABLE

Aqueous Solubility of Subject GI Contrast Media at 37°C at 6 hrs, at Equilibrium

| Compound No. | % Dissolved at pH 2 | at pH 8 |
|---|---|---|
| 3 | 1.7 | 2.0 |
| 5 | 28.5 | 31.6 |
| 7 | 5.1 | 3.8 |
| 8 | 18 | 24 |
| 11 | 13 | 13 |
| 14 | 14 | 15 |

EXAMPLE 15

Acylation of 5-amino-2,4,6-triiodo-isophthalic acid (17) to : 5-N-acrylamido-2,4,6-triiodo-isophthalic acid (18)

To a solution of the title compound (11, 111.8 g, 0.200 moles) in dry N,N-dimethylacetamide (400 ml) was added acryloyl chloride (20.3 ml, 0.250 moles) dropwise over 30 minutes. The mixture was stirred at room temperature for 3 days with further acryloyl chloride (20.3 ml, 0.250 moles) added. TLC indicated that no starting material remained. The solvent was removed under vacuum, the residue dissolved in water (250 ml) at 80°C, and the solution cooled. The resultant crystals were filtered, washed with water and dried in a vacuum oven to give 18 (134.7 g, ca. 95% yield after correction for water).

EXAMPLE 16

Synthesis of a Radiopaque Water-Soluble Copolymer with Low Iodine Content

A 12 L flask was charged with acrylamide (800 g, 11.25 moles), 5-N-acrylamido-2,4,6-triiodoisophthalic acid (18, 200 g, 0.33 moles), N,N'-methylene-bis-acrylamide (10 g, 0.065 moles) and isopropanol (5 L). The mixture was stirred until a uniform suspension was obtained. Ammonium persulfate (10 g, 0.044 moles) was added, and the mixture was heated at reflux for six hours. The reaction, homogeneous at first, became heterogenous towards the end.

The mixture was cooled to < 50°C, the solid was filtered and washed with isopropanol (1 L x 1), followed by methanol (2 L x 2), and dried in a vacuum oven at 80-100°C to give the crude polymer which was purified by resuspension in anhydrous methanol at 60°C (3 L ; repeated twice) and filtration. Drying and grinding gave 755 g polymer (ca. 75% yield).

UV-analysis indicated that the incorporation of the triiodo-moiety was 27% (w/w) corresponding to an iodine content of 16.8%. Following ultrafiltration a polymer was obtained with molecular weight > 100,000 and < 300,000 ; the pH of its aqueous solution was 2.1.

EXAMPLE 17

Synthesis of a Radiopaque Water-Soluble Copolymer with a High Iodine Content

In a 100 ml flask were combined acrylamide (2.0 g, 28 mMoles), 5-N-acrylamido-2,4,6-triiodoisophthalic acid (18, 8.0 g, 13 moles), N,N'-methylene-bis-acrylamide (0.1 g, 0.65 mMoles), benzoyl peroxide (0.1 g, 0.41 mMoles), and isopropanol (50 ml) and the mixture was heated at 80-90°C for 6 hours. The resultant insoluble polymer was filtered, washed with isopropanol (10 ml x 2), methanol (10 ml x 2), and dried. Resuspension in hot methanol, filtration, and drying reduced the residual triiodinated monomer to < 5% (size exclusion HPLC column). The polymer (5.49 g, yield ca. 54%) had a molecular weight distribution of 90% > 10,000 and 85% < 100,000. By uv-analysis the triiodomonomer incorporation was 92% (w/w), corresponding to iodine content of 57%.

The monomer was further reduced (to < 1%) by dissolving the polymer (1 g) in water (25 ml) and treating with Norit Ultra SX carbon (0.3 g) at 90°C for 1 hour.

EXAMPLE 18

Formulation of the Radiopaque Water-Soluble Copolymer For Computerized Tomography of the Gastrointestinal Tract

60 g copolymer of previous Example 16 was dissolved in $H_2O$ (800 ml) at 70-75°C, charcoal (Norit Ultra SX, 10 g) was added, and the mixture was refluxed for 1 hour. The carbon was removed by filtration. HPLC (size exclusion column) indicated that all monomers were removed by this treatment. The solution was ultrafiltered at 0.45 μm, and diluted to 1 L to achieve 9 mg I/ml a concentration suitable for gastrointestinal CT. $NaCA_2EDTA$ (0.1 mg/ml) was added and the solution was autoclaved at 120°C, for 20 minutes. The final pH was 2.12. The low free iodide levels (8 μg I/ml) and unchanged HPLC indicated pharmaceutically acceptable stability.

EXAMPLE 19

Synthesis of a Hydrolyzable Radiopaque Water-Soluble Copolymer

A mixture of acrylamide (7.1 g. 100 mMoles), 5-N-acrylamido-2,4,6-triiodo-isophthalic acid (18, 1.9 mMoles), ethylene glycol diacrylate (0.029 g, 0.17 mMoles), benzoyl peroxide (0.05 g, 0.21 mMoles) and t-butanol (50 ml) was heated at 80°C for 6 hours. The mixture became very thick by the end of the reaction. The polymer was filtered and washed with isopropanol (20 ml x 2), followed by methanol (20 ml x 2). Resuspension in hot methanol (repeated twice), followed by filtration, reduced the triiodo-monomer content in the polymer to 1-2% (size exclusion HPLC). Drying in a vacuum oven, followed by grinding gave a white powder (6.8 g, 75% yield). UV analysis indicated that triiodomoiety incorporation was 19.5% (w/w), corresponding to an iodine content of the polymer of 12.1%. Ultrafiltration analysis indicated that about 10% of the polymer had MW < 100,000, about 50% was between 100,000 and 1,000,000, and 40% was > 1,000,000 ; following preparative ultrafiltration a polymer with consistency of the latter two M.W. ranges was obtained.

EXAMPLE 20

Synthesis of a Highly Hydrophilic Radiopaque Water-Soluble Copolymer

A mixture of N-(2,3-dihydroxypropyl)acrylamide (8 g. 55 mMoles), 5-N-acrylamido-2,4,6-triiodoisophthalic acid (18, 2 g, 3.26 mMoles), N,N'-methylene-bis-acrylamide (0.1 g, 0.65 mMoles), ammonium persulfate (0.05 g, 0.22 mMoles) and isopropanol (75 ml) was heated for 6 hours at 75-85°C. The suspension was cooled, the polymer filtered and washed with isopropanol (25 ml x 2) followed by methanol (25 ml x 3). Vacuum drying at 70-80°C overnight gave 6.1 g polymer containing ca. 2% monomers (detected by size exclusion HPLC). The monomers were conveniently removed by treatment of an aqueous solution of the polymer with Norit Ultra SX carbon. The incorporation of the triiodo-moiety was ca. 23% (w/w, uv-analysis), corresponding to an iodine content of 14.3% (w/w).

EXAMPLE 21

Synthesis of a Highly Hydrophilic Radiopaque Water-Soluble Hydrolyzable Copolymer

A mixture of N-(2,3-dihydroxypropyl)acrylamide (8 g, 55 mMoles), 5-N-acrylamido-2,4,6-triiodo-isophthalic acid (18, 2 g, 3.26 mMoles), ethylene glycol diacrylate (0.1 g, 0.59 mMoles), benzoyl peroxide (0.05 g, 0.21 mMoles) and t-butanol (75 ml) was heated to reflux for 6 hours. The suspension was cooled, the sold filtered and washed with methanol (50 ml x 3). Following drying in a vacuum oven at 60°C, 7.2 g polymer was obtained. Incorporation of the triiodo-moiety was ca. 21% (w/w), corresponding to an iodine content of 13% (w/w).

The subject monomeric compounds for plain radiography are formulated as a suspension and the polymeric compounds as solutions prior to oral or rectal administration. When injected intraperitoneally into normal rats weighing 80-100 grams, in a dose of 150 mg I, they typically are resorbed within 24 to 48 hours. Upon histological examination, the peritoneum did not show any sign of adverse reactions. The monomeric compounds are, upon resorption by the peritoneum, taken into the circulation and excreted into the urine like other radiographic contrast medial ; the esters are not appreciably hydrolyzed. The polymeric compounds are also resorbed by the peritoneum and deposited into the reticuloendothelial system. When hydrolyzable linkages are provided, the compounds are excreted via the urine.

In CT gastrointestinal application, all disadvantages of current water-soluble media or barium are eliminated by the use of hypo- or isoosmolar highly hydrophilic and nontoxic copolymers containing a radio-diagnostic moiety. Furthermore, when hydrolyzable linkages are incorporated into such copolymers, such as esters, which upon esterase facilitated hydrolysis produce fragments not exceeding 50,000 daltons, the copolymers are rapidly biodegraded. This adds additional safety to their otherwise low toxicity, should they, through a perforation within the gastrointestinal tract, leak into the peritoneal cavity, or accidentally enter the lymphatic or cardiovascular system. The basic profile of the new copolymers is shown in Table 1.

TABLE 1

| | Compound Example 16 | Compound Example 17 | Compound Example 19 |
|---|---|---|---|
| Estimated Molecular Weight Range | 5,000-300,000 | 5,000-100,000 | ≥100,000 |
| Osmolality (mOsm) | 210 (30 mg I/ml)<br>62 (9 mg I/ml) | 285 (103 mg I/ml)<br>50 (10 mg I/ml) | 52 (15 mg I/ml)<br>35 (10 mg I/ml) |
| % Iodine (w/w) | 16.8 | 52 | 19.5 |
| Approx. range, IV $LD_{50}$ (male mice) (g I/kg BW) | 1.7-2.0 | 2-3 | N/A |
| Approx. range, IV $LD_{50}$ (male rats) (g I/kg BW) | 1.5-2.0 | 2-3.5 | N/A |
| Histology, 5 days after intraperitoneal injection (rats) | | No changes observed | |

The preliminary tests of a diagnostically useful concentration of 10 mg I/ml of the copolymer according to Example 16 and 18 in human CT application proved the material coats the gastrointestinal wall, is uniformly dispersed, gives no imaging artifacts, and delineates the entire tract with the same intensity of contrast throughout. The material also did not precipitate but seeped along the intestinal wall even in the presence of intestinal contents. When compared to the standard barium suspensions or water-soluble contrast media currently used in clinical practice, the CT image quality obtained with this novel class of media is not of quantitatively, but qualitatively improved difference.

Also, when the copolymer according to Example 15, 17 was injected into rate, in a 1 ml dose (50 mg I/ml), it provided an opacification persisting for 4 hours of the heart major vessels and kidneys by computerized tomography ; it has no apparent toxicity during the 3-week survival, and the histology of the key organs was normal. When compared to the standard water-soluble contrast media used in clinical vascular practice, the new copolymers provide a new type of imaging. The standard media diffuse from the vascular bed within seconds, thus providing only a poor contrast and few images in a given region. Also, they have to be administered in high doses ranging from 100 to 250 ml of 300 mg I/ml solutions, a dose which usually elicits adverse effects. The new copolymers allow for a long time selective imaging of the entire blood pool by CT and they are toxicologically superior to the current media : with the clinical dose of 10 mg I/ml, the new copolymers provide a large margin of safety.

It is evident from the above results, that the subject compounds provide for many of the desirable properties of a contrast medium material for the GI tract. The compositions have low toxicity, good physiological properties and can be economically and efficiently produced from readily available starting materials.

The resulting compounds have either no taste or a pleasant taste resembling soft drinks, and can have a pH either adjusted with NaOH or left acidic. Thus, no taste modifiers are necessary. They are formulated only with water and small amounts of Ca,Na EDTA such as 0.1 mg EDTA if autoclaving is desired. Alternatively, they can be dispensed as a powder and diluted in water prior to the examination.

The highly hydrophilic polyacrylamide hydrolyzable radiopaque copolymers also find use in CT for visualization of other body cavities than the GI tract ; they can also visualize the cardiovascular system, thus providing a blood-pool contrast agent.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

**Claims**

1. A compound useful as a contrast medium, said compound having the following formula :

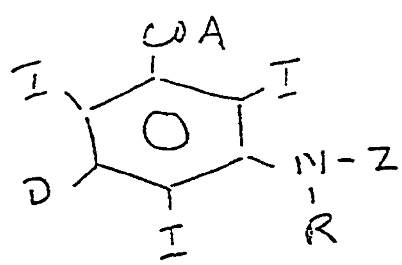

wherein :

Z is acetyl, an addition polymerizable acrylyl group, or a monomeric unit of an acrylyl polymer, wherein said acrylyl group has from 0 to 1 substituent at the α carbon atom, said substituent being alkyl, hydroxyalkyl, carboxamidoalkyl, carboxyalkyl or a monomeric acrylyl group, said substituent being of from 1 to 5 carbon atoms ;

when Z is acetyl :

R is hydrogen, lower alkyl of from 1 to 6 carbon atoms or hydroxy- or polyhydroxyalkyl of from 2 to 6 carbon atoms ;

D is —NR'$COCH_3$, $CONHCH_2CH_2OH$ or $CONHCH_3$ ; and

R and R' are H, alkyl of from 1 to 6 carbon atoms or hydroxy- or polyhydroxyalkyl of from 2 to 6 carbon atoms, wherein R and R' may be same or different ;

A is alkoxy of from 1 to 4 carbon atoms or hydroxy- or polyhydroxyalkyl of from 1 to 4 carbons ;
when Z is an addition polymerizable acrylyl group wherein the carbon is unsubstituted or substituted with alkyl, hydroxyalkyl, carboxamido alkyl or carboxyalkyl or a monomeric unit of an acrylyl polymer :
R is hydrogen, alkyl or hydroxyalkyl ;
D is carboxyl, carboxylate with a physiologically acceptable counter ion or carboxamide, where the nitrogen may have from 0 to 2 substituents, which substituents are alkyl of from 1 to 4 carbon atoms, or hydroxylalkyl of from 2 to 4 carbon atoms and 1 to 3 hydroxyl groups, with any remaining positions occupied by hydrogens ;
A is hydroxyl, a physiologically acceptable salt thereof, or amino, where the nitrogen may have from 0 to 2 substituents, which substituents are alkyl of from 1 to 4 carbon atoms, hydroxyalkyl of from 2 to 4 carbon atoms bearing 1 to 3 hydroxyl groups, any remaining positions being occupied by hydrogen.

2. A compound according to Claim 1, wherein Z is acetyl.

3. A compound according to Claim 1, wherein Z is an acryl group.

4. A compound according to Claim 1, wherein Z is a monomeric unit of an acryl addition polymer.

5. A compound according to claim 1 or 2 having the following formula :

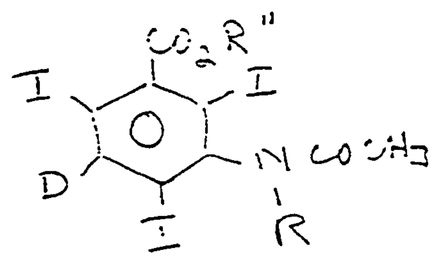

wherein :
R″ is lower alkyl of from 1 to 3 carbon atoms or hydroxy- or di-or polyhydroxyalkyl of from 2 to 3 carbon atoms ;
D is —NR′$COCH_3$, $CONHCH_2CH_2OH$ or $CONHCH_3$ ; and
R and R′ are H, alkyl of from 1 to 4 carbon atoms or hydroxy- or di-or polyhydroxyalkyl of from 2 to 4 carbon atoms, wherein R, R′ and R″ may be the same or different.

6. A compound according to claim 5 having the following formula :

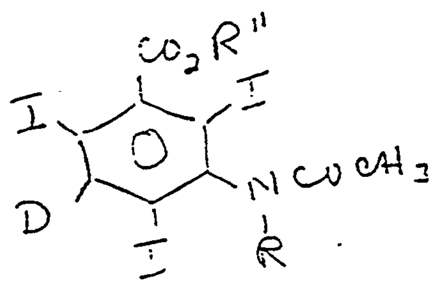

wherein :
R″ is trihydroxybutyl, dihydroxypropyl, 2-hydroxyethyl or methyl ;
D is —NR′$COCH_3$, $CONHCH_2CH_2OH$ or $CONHCH_3$ ; and
R and R′ are H, or come within the definition of R″, wherein R, R′ and R″ may be same or different.

7. A compound useful to prepare a polymer as contrast medium, said compound having the following formula:

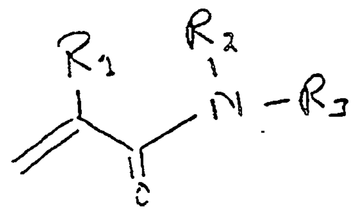

wherein :

R1 is hydrogen, alkyl, hydroxyalkyl, carboxamidoalkyl, carboxyalkyl or monomeric acrylyl group, said substituent being of from 1 to 5 carbon atoms ;

R2 is hydrogen, alkyl of from 1 to 3 carbon atoms or hydroxyalkyl of from 2 to 3 carbon atoms and 1 to 2 hydroxyl groups ;

R3 is of the formula :

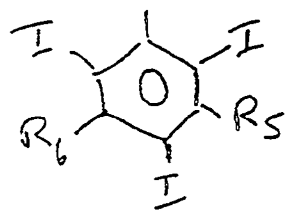

wherein :

R4 and R5 are the same or different and are non-oxo-carbonyl or amino, at least one being non-oxo-carbonyl, where the non-oxo-carbonyl group is a carboxylic acid, its physiologically acceptable salt or carboxamide, wherein the nitrogen has from 0 to 2 substituents, which substituents are alkyl of from 1 to 4 carbon atoms or hydroxyalkyl of from 2 to 4 carbon atoms and 1 to 3 hydroxyl groups.

**8.** A copolymer comprising a monomer according to claim 7 and an acrylic acid monomer having from 0 to 1 substituent at the α-position, wherein said acrylic acid monomer is the acid, physiologically acceptable salt, ester, amide or substituted amide.

**9.** A copolymer according to claim 8, further comprising a diacrylic acid, wherein the acrylic acids are joined by a physiologically hydrolyzable linking group.

**10.** A contrast medium formulation comprising from 0.5 to 80 weight percent of a compound according to any one of claims 1 to 6 in a physiologically acceptable aqueous medium.

**11.** A compound according to any one of claims 1 to 6 or a formulation according to claim 10 for use as a contrast medium.

**12.** Use of a compound according to any one of claims 1 to 6 in the manufacture of a contrast medium.

**Claims for the following Contracting State : ES**

**1.** A process for preparing a contrast medium, comprising using a compound having the following formula :

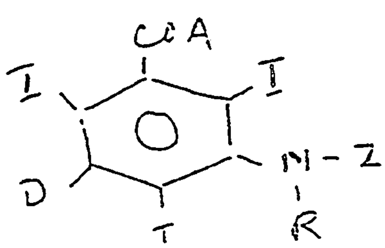

wherein :

Z is acetyl, an addition polymerizable acrylyl group, or a monomeric unit of an acrylyl polymer, wherein said acrylyl group has from 0 to 1 substituent at the $\alpha$ carbon atom, said substituent being alkyl, hydroxyalkyl, carboxamidoalkyl, carboxyalkyl or a monomeric acrylyl group, said substituent being of from 1 to 5 carbon atoms ;

when Z is acetyl :

R is hydrogen, lower alkyl of from 1 to 6 carbon atoms or hydroxy- or polyhydroxyalkyl of from 2 to 6 carbon atoms ;

D is —$NR'COCH_3$, $CONHCH_2CH_2OH$ or $CONHCH_3$ ; and

R and R′ are H, alkyl of from 1 to 6 carbon atoms or hydroxy- or polyhydroxyalkyl of from 2 to 6 carbon atoms, wherein R and R′ may be same or different ;

A is alkoxy of from 1 to 4 carbon atoms or hydroxy- or polyhydroxyalkyl of from 1 to 4 carbons ;

When Z is an addition polymerizable acrylyl group wherein the carbon is unsubstituted or substituted with alkyl, hydroxyalkyl, carboxamido alkyl or carboxyalkyl or a monomeric unit of an acrylyl polymer :

R is hydrogen, alkyl or hydroxyalkyl ;

D is carboxyl, carboxylate with a physiologically acceptable counter ion or carboxamide, where the nitrogen may have from 0 to 2 substituents, which substituents are alkyl of from 1 to 4 carbon atoms, or hydroxylalkyl of from 2 to 4 carbon atoms and 1 to 3 hydroxyl groups, with any remaining positions occupied by hydrogens ;

A is hydroxyl, a physiologically acceptable salt thereof, or amino, where the nitrogen may have from 0 to 2 substituents, which substituents are alkyl of from 1 to 4 carbon atoms, hydroxyalkyl of from 2 to 4 carbon atoms bearing 1 to 3 hydroxyl groups, any remaining positions being occupied by hydrogen.

2. A process according to Claim 1, wherein Z is acetyl.

3. A process according to Claim 1, wherein Z is an acryl group.

4. A process according to Claim 1, wherein Z is a monomeric unit of an acryl addition polymer.

5. A process according to claim 1 or 2, the compound having the following formula :

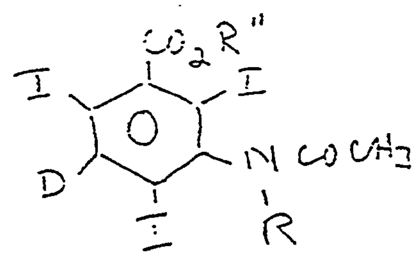

wherein :

R″ is lower alkyl of from 1 to 3 carbon atoms or hydroxy- or di-or polyhydroxyalkyl of from 2 to 3 carbon atoms ;

D is —$NR'COCH_3$, $CONHCH_2OH$ or $CONHCH_3$ ; and

R and R′ are H, alkyl of from 1 to 4 carbon atoms or hydroxy- or di-or polyhydroxyalkyl of from 2 to 4 carbon atoms, wherein R, R′ and R″ may be the same or different.

6. A process according to claim 5, the compound having the following formula :

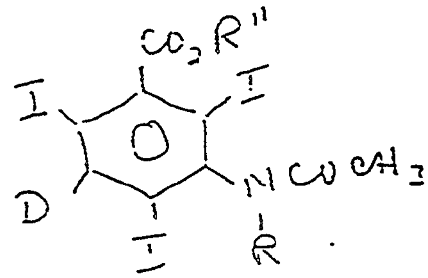

wherein :

R″ is trihydroxybutyl, dihydroxypropyl, 2-hydroxyethyl or methyl ;
D is —NR′COCH3, CONHCH2CH2OH or COCHCH3 ; and
R an R′ are H, or come within the definition of R″, wherein R, R′ and R″ may be same or different.

7. A process for preparing a contrast medium, the process including using a compound having the following formula :

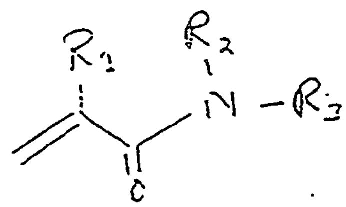

31

wherein :

R1 is hydrogen, alkyl, hydroxyalkyl, carboxamidoalkyl, carboxyalkyl or monomeric acrylyl group, said substituent being of from 1 to 5 carbon atoms ;
R2 is hydrogen, alkyl of from 1 to 3 carbon atoms or hydroxyalkyl of from 2 to 3 carbon atoms and 1 to 2 hydroxyl groups ;
R3 is of the formula :

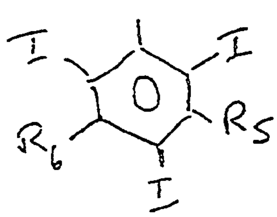

wherein :

R4 and R5 are the same or different and are non-oxo-carbonyl or amino, at least one being non-oxo-carbonyl, where the non-oxo-carbonyl group is a carboxylic acid, its physiologically acceptable salt or carboxamide, wherein the nitrogen has from 0 to 2 substituents, which substituents are alkyl of from 1 to 4 carbon atoms or hydroxyalkyl of from 2 to 4 carbon atoms and 1 to 3 hydroxyl groups.

8. A process according to claim 7 wherein there is an acrylic acid monomer having from 0 to 1 substituent at the α-position, wherein said acrylic acid monomer is the acid, physiologically acceptable salt, ester, amide or substituted amide.

9. A process according to claim 8, wherein the compound further comprises a diacrylic acid, wherein the acrylic acids are joined by a physiologically hydrolyzable linking group.

10. A process contrast according to any one of the preceding claims comprising using from 0.5 to 80 weight percent-of-a compound in a physiologically acceptable aqueous medium.

11. Use of a compound according to any one of claims 1 to 10 in the manufacture of a contrast medium.

**Claims for the following Contracting State : GR**

1. A process for preparing a contrast medium, comprising using a compound having the following formula :

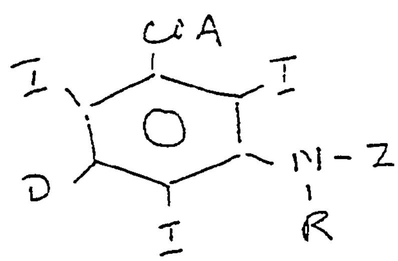

wherein :

Z is acetyl, an addition polymerizable acrylyl group, or a monomeric unit of an acrylyl polymer, wherein said acrylyl group has from 0 to 1 substituent at the α carbon atom, said substituent being alkyl, hydroxyalkyl, carboxamidoalkyl, carboxyalkyl or a monomeric acrylyl group, said substituent being of from 1 to 5 carbon atoms ;

when Z is acetyl :

R is hydrogen, lower alkyl of from 1 to 6 carbon atoms or hydroxy- or polyhydroxyalkyl of from 2 to 6 carbon atoms ;

D is —NR'$COCH_3$, $CONHCH_2CH_2OH$ or $CONHCH_3$ ; and

R and R' are H, alkyl of from 1 to 6 carbon atoms or hydroxy- or polyhydroxyalkyl of from 2 to 6 carbon atoms, wherein R and R' may be same or different ;

A is alkoxy of from 1 to 4 carbon atoms or hydroxy- or polyhydroxyalkyl of from 1 to 4 carbons ;

when Z is an addition polymerizable acrylyl group wherein the carbon is unsubstituted or substituted with alkyl, hydroxyalkyl, carboxamido alkyl or carboxyalkyl or a monomeric unit of an acrylyl polymer :

R is hydrogen, alkyl or hydroxyalkyl ;

D is carboxyl, carboxylate with a physiologically acceptable counter ion or carboxamide, where the nitrogen may have from 0 to 2 substituents, which substituents are alkyl of from 1 to 4 carbon atoms, or hydroxylalkyl of from 2 to 4 carbon atoms and 1 to 3 hydroxyl groups, with any remaining positions occupied by hydrogens ;

A is hydroxyl, a physiologically acceptable salt thereof, or amino, where the nitrogen may have from 0 to 2 substituents, which substituents are alkyl of from 1 to 4 carbon atoms, hydroxyalkyl of from 2 to 4 carbon atoms bearing 1 to 3 hydroxyl groups, any remaining positions being occupied by hydrogen.

2. A process according to Claim 1, wherein Z is acetyl.

3. A process according to Claim 1, wherein Z is an acryl group.

4. A process according to Claim 1, wherein Z is a monomeric unit of an acryl addition polymer.

5. A process according to claim 1 or 2, the compound having the following formula :

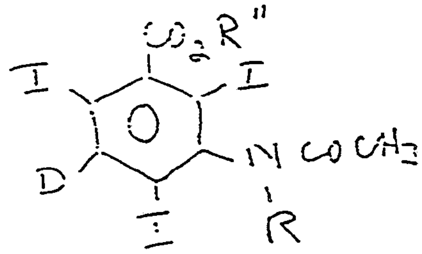

wherein :

R″ is lower alkyl of from 1 to 3 carbon atoms or hydroxy- or di-or polyhydroxyalkyl of from 2 to 3 carbon atoms ;

D is —NR′$COCH_3$, $CONHCH_2CH_2OH$ or $CONHCH_3$ ; and

R and R′ are H, alkyl of from 1 to 4 carbon atoms or hydroxy- or di-or polyhydroxyalkyl of from 2 to 4 carbon atoms, wherein R, R′ and R″ may be the same or different.

6. A process according to claim 5, the compound having the following formula :

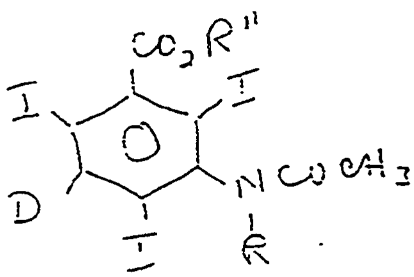

wherein :

R″ is trihydroxybutyl, dihydroxypropyl, 2-hydroxyethyl or methyl ;

D is —NR′COCH3, CONHCH2CH2OH or CONHCH3 ; and

R and R′ are H, or come within the definition of R″, wherein R, R′ and R″ may be same or different.

7. A process for preparing a contrast medium, the process including using a compound having the following formula :

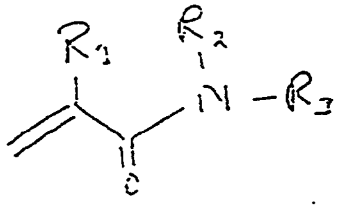

wherein :

R1 is hydrogen, alkyl, hydroxyalkyl, carboxamidoalkyl, carboxyalkyl or monomeric acrylyl group, said substituent being of from 1 to 5 carbon atoms ;

R2 is hydrogen, alkyl of from 1 to 3 carbon atoms or hydroxyalkyl of from 2 to 3 carbon atoms and 1 to 2 hydroxyl groups ;

R3 is of the formula :

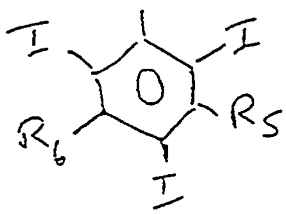

wherein :

R4 and R5 are the same or different and are non-oxo-carbonyl or amino, at least one being non-oxo-carbonyl, where the non-oxo-carbonyl group is a carboxylic acid, its physiologically acceptable salt or carboxamide, wherein the nitrogen has from 0 to 2 substituents, which substituents are alkyl of from 1 to 4 carbon atoms or hydroxyalkyl of from 2 to 4 carbon atoms and 1 to 3 hydroxyl groups.

**8.** A process according to claim 7 wherein there is an acrylic acid monomer having from 0 to 1 substituent at the $\alpha$-position, wherein said acrylic acid monomer is the acid, physiologically acceptable salt, ester amide or substituted amide.

**9.** A process according to claim 8, wherein the compound further comprises a diacrylic acid, wherein the acrylic acids are joined by a physiologically hydrolyzable linking group.

**10.** A process contrast according to any one of the preceding claims comprising using from 0.5 to 80 weight percent-of-a compound in a physiologically acceptable aqueous medium.

**11.** Use of a compound according to any one of claims 1 to 10 in the manufacture of a contrast medium.

European Patent Office

# EUROPEAN SEARCH REPORT

Application Number

EP 90313363.5

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.5) |
|---|---|---|---|
| D,A | DE - A1 - 2 428 140<br>(BEECHAM)<br>* Claims *<br>-- | 1,2,5,<br>12 | C 07 C 233/53<br>C 07 C 235/16<br>C 07 C 237/46<br>A 61 K 49/04 |
| A | US - A - 4 567 034<br>(I. CHARLES et al.)<br>* Claims *<br>-- | 1,2,5,<br>12 | |
| A | DE - A1 - 3 428 265<br>(SCHERING)<br>* Totality *<br>-- | 1,7,12 | |
| D,A | DIE MARKROMOLEKULARE CHEMIE, RAPID COMMUNICATIONS, vol. 6, 1985, Hüthig & Wepf Verlag, Basel, Heidelberg, New York<br>E. BROWN et al. "Syntheses and copolymerizations of new water-soluble polyiodinated acrylic monomers"<br>* Pages 503-507 *<br>-- | 1,3,4,<br>7,12 | TECHNICAL FIELDS SEARCHED (Int. Cl.5) |
| D,A | BULLETIN DE LA SOCIETE CHIMIQUE DE FRANCE, vol. 4, Juli-August 1986<br>pages 669-677<br>* Page 670 *<br>---- | 1,3,7,<br>12 | C 07 C 233/00<br>C 07 C 235/00<br>C 07 C 237/00<br>A 61 K 49/00 |

The present search report has been drawn up for all claims

| Place of search | Date of completion of the search | Examiner |
|---|---|---|
| VIENNA | 15-03-1991 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)